# EUROPEAN PATENT SPECIFICATION

(11) **EP 1 958 591 B1**
(45) Date of publication and mention of the grant of the patent: **11.05.2016**
(21) Application number: 08250500.9
(22) Date of filing: 12.02.2008
(51) Int. Cl.: A61F 2/08, G01B 3/11

(54) **Intraarticular graft length gauge**
Messgerät zur Längenbestimmung von Sehnen innerhalb von Gelenken
Jauge de longueur pour une greffe intra-articulaire

(30) Priority: 13.02.2007 US 900988 P
(43) Date of publication of application: 20.08.2008
(73) Proprietor: Arthrex, Inc., Naples, FL 34108-1945 (US)
(72) Inventor: Jolly, Jacob, Naples FL 34110-2880 (US); Albertorio, Ricardo, Naples 34119-4107 Florida (US); Karnes, Joshua G., Naples 34109 Florida (US); Schmieding, Reinhold, Naples 34108 Florida (US)
(74) Representative: Carpmael, Robert Maurice Charles

(56) References cited:
- WO-A1-96/03086
- DE-A1- 3 542 460
- DE-U1- 9 002 844
- DE-U1- 9 005 819
- GB-A- 181 606
- US-A1- 2002 019 649
- US-A1- 2007 016 208

## Description

### FIELD OF THE INVENTION

The present invention relates to the field of surgery and, more particularly, to an apparatus for improved graft fixation in ACL reconstructive surgeries.

### BACKGROUND OF THE INVENTION

Reconstructive surgeries, particularly anterior cruciate ligament (ACL) reconstruction, are well-known in the art. In general, these methods of tenodesis involve drilling a tunnel through the tibia, drilling a closed tunnel (socket) into the femur, inserting a substitute ACL graft into the tunnels, and securing the grafts to the walls of the tibial and femoral tunnels using interference screws or the like.

Recently, an "all-inside" ACL technique has been developed, wherein two closed tunnels (sockets) are drilled, one through the tibia and one through the femur. The femoral socket is formed by using a retrograde drill device provided with a retrograde drill cutter detachable from a retrograde drill guide pin, in the manner described in U.S. Patent Application Publication No. 2004/0199166. The tibial tunnel or socket may be formed by the retrograde drill method or by a conventional method, and may be carried out before or after the formation of the femoral socket.

Graft length determination in the above-described "all-inside" retrograde drill technique is difficult, as adjustment of the length of the graft (by cutting the graft at the end of the tibial tunnel, for example) is impossible since the tunnels are not drilled from the outer cortex (as in a conventional ACL technique). It is desirable, therefore, to use a graft with a predetermined yet exact length to be secured intraarticularly within the sockets (tunnels). A graft with a predetermined, exact length would eliminate any movement of the graft from side to side (increasing the life of the ACL repair) and would allow proper tensioning of the graft subsequent to its fixation. A method for determining the appropriate length of a graft for positioning of the graft intraarticularly is also desirable.

### SUMMARY OF THE INVENTION

The present invention seeks to provide a technique and reconstruction system for ligament repair by determining the exact length of the graft to be secured within a first tunnel or socket (for example, a tibial tunnel) and a second tunnel or socket (for example, a femoral tunnel) for improved graft fixation.

The system of the present invention comprises a measuring device as defined in claim 1 for determining the appropriate length of the graft (soft tissue graft or BTB graft, for example) by determining the length of a first socket in a first bone region (for example, a tibial socket), the length of a second socket in a second bone region (for example, a femoral socket), and the length of the intraarticular joint space between the two sockets in the two bone regions (for example, tibia and femur). The measuring device consists of a flexible member (for example, suture) with two fixation devices attached to the flexible member, the fixation devices being selected from the group consisting of a sliding knot, a washer and a button.

PCT Publication WO96/03086 discloses a protective tube or sheath for surrounding the replacement ligament, aiding in locating and protecting the replacement ligament.

Other features and advantages of the present invention will become apparent from the following description of the invention which refers to the accompanying drawings.

### BRIEF DESCRIPTION OF THE DRAWINGS

Figure 1 illustrates an exemplary suture-button construct for graft length determination according to the present invention.
Figure 2 illustrates a measuring device comprising a suture and two sliding knots for graft length determination according to an embodiment of the present invention.
Figures 3-5 illustrate various steps of a method of ACL reconstruction employing the measuring device of Figure 2 and according to an exemplary embodiment of the present invention.
Figures 6 and 7 illustrate a continuous loop/button construct used for fixating a graft with a length predetermined according to an embodiment of the present invention and used during ACL reconstruction.

### DETAILED DESCRIPTION OF AN EMBODIMENT THE INVENTION

The present invention provides a technique and reconstruction system for ligament or tendon repair. The system of embodiments of the present invention comprise a measuring device designed to determine the appropriate graft length to achieve strong graft fixation and tensioning during ACL reconstruction. The measuring device allows proper tensioning and fixation of the graft in an "all-inside" ACL technique, wherein use of special measurement devices (such as a drill pin provided with laser marks to measure the distance from the femoral notch to the outer cortex, by itself or in conjunction with a depth guide, for example) is not possible due to the way the femoral and tibial tunnels are formed.

The system of embodiments of the present invention comprise a measuring device for determining the appropriate length of the graft (for example, soft tissue graft or BTB graft) by determining the length of the first socket in a first bone region (for example, the tibial socket), the length of the second socket in a second bone region (for example, the femoral socket), and the length of the intraarticular joint space between the two bone regions (for example, tibia and femur).

According to one example, the measuring device is a miniature version of the suture-button construct described in U.S. Appl. Publ. No. 2007/0016208. In an embodiment, the measuring device consists of a flexible member (for example, suture) with two sliding knots. In alternative embodiments, the measuring device comprises of a flexible member (for example, suture) with two fixation device selected from the group consisting of a sliding knot, a washer and a button.

The specification also discloses a method of ACL reconstruction using a graft having a length that is predetermined by measuring, intraarticularly, the length of the tibial and femoral sockets plus the intraarticular space between tibia and femur. The method of ACL reconstruction comprises, for example, the steps of: (i) drilling at least a femoral tunnel or socket and at least a tibial tunnel or socket; (ii) determining the entire length of the sockets plus the intraarticular space by employing a miniature suture-button construct and/or a measuring device consisting of a flexible member (for example, suture) with at least one fixation device selected from the group consisting of a sliding knot, a washer and a button; (iii) determining the length of the graft (soft tissue graft or BTB graft) based on the entire length of the sockets plus the intraarticular space between them; and (iv) securing the graft within the femoral and tibial tunnels (sockets).

The specification also discloses a method of ACL reconstruction that comprises the steps of: (i) drilling at least a femoral and tibial tunnel or socket using a retrograde drill technique; (ii) determining a first distance D₁ (which is the length of the tibial and femoral sockets plus the intraarticular space) by employing a miniature suture-button construct and/or a measuring device consisting of a flexible member (for example, suture) with at least one fixation device selected from the group consisting of a sliding knot, a washer and a button; (iii) determining a distance D₂ (which is equal to D₁ minus about 5 to about 10mm); (iv) providing a graft (soft tissue graft or BTB graft) and cutting the graft to a length about equal to D₂; (v) securing the graft (soft tissue graft or BTB graft) to a continuous loop/button construct comprising a button with an oblong configuration and provided with an inside eyelet that allows the passage of the continuous loop, preferably a suture loop; (vi) passing the graft with the button through the femoral tunnel; and (vii) securing the button to the femoral cortex once the button exits the femoral socket.

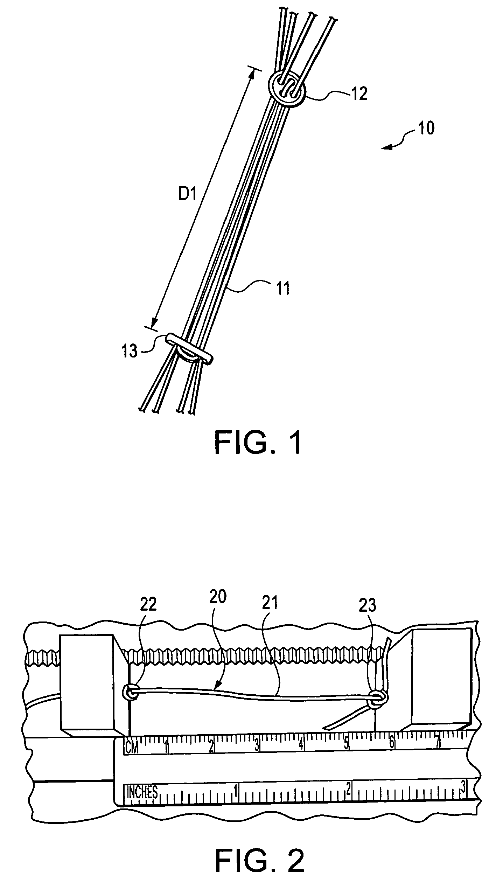

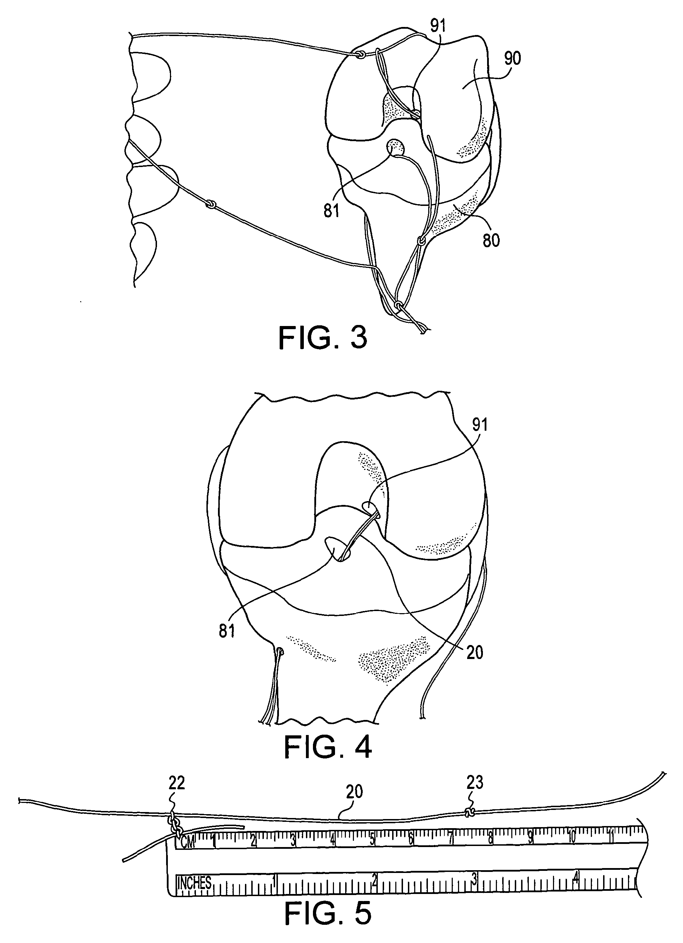

Referring now to the drawings, where like elements are designated by like reference numerals, Figures 1 and 2 illustrate exemplary embodiments of measuring devices 10, 20 of the present invention. Figures 3-5 illustrate various steps of a method of ACL reconstruction employing the measuring device 20 of Figure 2.

Figure 1 illustrates a miniature suture-button construct 10, a miniature version of the device described in U.S. Appl. Publ. No. 2007/0016208. Construct 10 may comprise, for example, two buttons 12, 13 (Figure 1) each having an oblong body defining first and second apertures, or a button and a washer, among other configurations.

The suture-button construct 10 may comprise a high strength suture, such as Arthrex FiberWire® or lasso wire 11. Exemplary steps for employing the suture-button construct 10 to determine the appropriate graft length are as follows: Two ends of the suture are passed through one button so the loop is at the bottom of the first button. This allows tying of passing sutures to graft and will bottom out the first button at the end of the loop. The free ends of the suture are passed through the eyelets of another round button, loop wire or suture and looped twice, to reduce slippage. Another loop is created on the other end of the suture tails to tie graft passing sutures. The construct is pulled into the joint with the graft passing sutures, bottoming out with the fixed button first (tibia), then the other end is tensioned and bottomed out until the suture or wire between the sockets is tight. The midsection of the gauge (of the suture strand 11) is grabbed through a cannula with a grasper and pulled out until the buttons are in the cannula to avoid slippage on removal through the anteriomedial portal. The distance D₁ between the two buttons 12, 13 may be exactly the length of the graft since the greater diameter of the tendons positioned in the middle of the tunnels may end up giving the 5-10 mm tensioning distance, since the gauge 11 extends from rim to rim versus center to center with a tight fitting graft. Alternatively, the length of the graft may be determined based on the distance D₁ between the two buttons minus about 5 to about 10 mm, to allow tensioning of the graft upon insertion within the bone tunnels/sockets.

Subsequent to the determination of distance D₁, grafts (for example, allografts) may be prepared for fixation using the Arthrex RetroButton™ (continuous suture loop/button construct) technique, for example, and extra long Fiberloop™ stitching, to speed preparation. The construct 10 is positioned on the graft and the final few stitches of the Fiberloop™ removed, to shorten to the exact length needed. In this manner, preparation of the graft with just drilling and measuring is achieved.

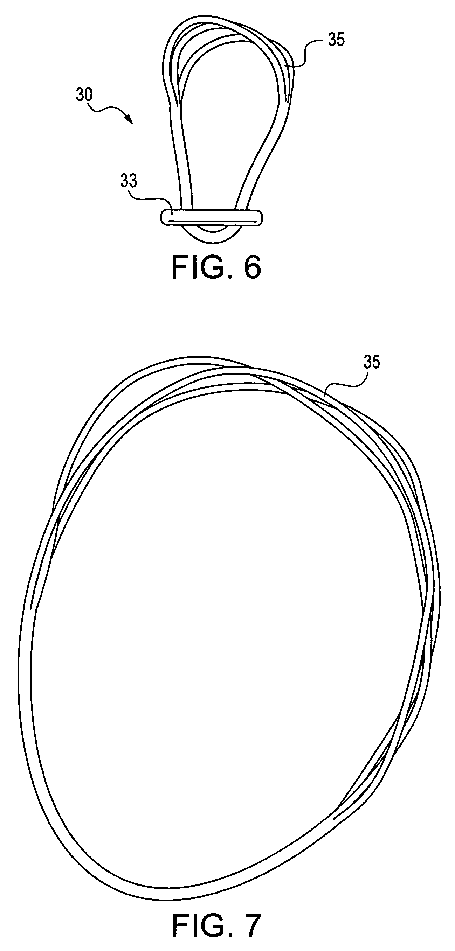

Once the length of the graft is adequately determined, preparation of the allograft may be conducted by employing a continuous loop/button construct 30 (Figures 6 and 7) provided with a button 33, preferably of titanium alloy, and a continuous loop 35 attached to the button 33. The button 33 may have an oblong configuration and a width that is preferably less than about 1mm narrower than the width of the drill hole through which the button 33 is inserted and subsequently passed through. The button 33 is provided with an inside eyelet that allows the passage of the continuous loop. In an exemplary embodiment, the suture loop 35 may be a single high strength suture such as FiberWire^{®} suture, sold by Arthrex, Inc. of Naples, Florida, and described in U.S. Patent No. 6,716,234. In another exemplary embodiment, the continuous loop 35 may be formed of a plurality of suture strands configured to separate from a single strand to a plurality of strands in a continuous loop.

In an exemplary embodiment, the continuous loop/button construct 30 is used to secure a soft tissue graft (measured according to an embodiment of the present invention) in a bone socket in a retrograde manner, for example. According to another exemplary embodiment, the continuous loop/button construct 30 is used to secure a bone-to-bone (BTB) graft (measured according to an embodiment of the present invention) in a femoral tunnel or socket in a retrograde manner, for example.

In these particular and only exemplary embodiments, a method of ACL reconstruction using the continuous loop/button construct and graft (measured according to an embodiment of the present invention) comprises, for example, the steps of: (i) drilling at least one femoral tunnel or socket, and at least one tibial tunnel or socket, wherein the drilling of the at least one femoral tunnel may be conducted using a retrograde drill cutter which is inserted in a retrograde manner through the femur; (ii) determining the entire length of the sockets plus the intraarticular space by employing the miniature suture-button construct 10; (iii) determining the length of the graft (soft tissue graft or BTB graft) based on the entire length of the sockets plus the intraarticular space between them, as measured by the suture-button construct 10; and (iv) securing the graft within the femoral and tibial tunnels (sockets).

The at least one femoral socket may be prepared by employing a retrograde drill device provided with a retrograde drill cutter detachable from a retrograde drill guide pin, in the manner described in U.S. Patent Application Publication No. 2004/0199166. As described in U.S. Patent Application Publication No. 2004/0199166, a retrograde drill device for ACL reconstruction is provided with a retrograde drill cutter detachable from a retrograde drill guide pin. The retrograde drill cutter is inserted in a retrograde manner through the femur by employing a retrograde drill guide pin provided with depth markings. Formation of the tibial tunnel or socket by the method described above or by a conventional method may be carried out before or after the formation of the femoral socket.

Once the femoral and tibial tunnels or sockets have been completed, graft insertion and fixation may be subsequently carried out. According to an exemplary embodiment of the present invention, the graft (which may be a soft tissue graft) is folded in half over the loop of the button 30 and tension is applied. Subsequently, passing sutures are pulled and the graft is passed into the femoral tunnel or socket. When the graft reaches the opening of the femoral socket or tunnel on the femoral cortex, a slight popping sensation may be felt as the button exits and begins to flip horizontally on the femoral cortex. Distal traction on the graft and release of the passing sutures facilitate complete deployment of the button. The passing suture may be removed and tibial fixation may be completed.

The exemplary techniques of ACL reconstruction detailed above are further described below with reference to Figures 3-5 and using a measuring device 20 which consists of a flexible member 21 (for example, suture) with two sliding knots 22, 23. In alternative embodiments, the measuring device comprises a flexible member (for example, suture) with two fixation devices selected from the group consisting of a sliding knot, a washer and a button. The flexible member may be a suture strand, for example, a single high strength suture such as FiberWire^{®} suture, sold by Arthrex, Inc. of Naples, Florida, and described in U.S. Patent No. 6,716,234. In additional examples, the flexible member may be formed of a plurality of flexible strands, at least one of the strands being a suture strand.

The measuring device 20 is employed to determine the appropriate graft length, in a manner similar to the one described above with reference to the suture-button construct 10. Specifically, an exemplary method of determining the appropriate length of a graft to be secured within a first opening/tunnel within a first bone region and within a second opening/tunnel within a second bone region, comprises the steps of: (i) drilling a femoral tunnel or socket 91 using, for example, a retrograde drill cutter which is inserted in a retrograde manner through femur 90; (ii) drilling a tibial tunnel or socket 81 through tibia 80; (iii) determining the entire length of the sockets 81, 91 plus the length of the intraarticular space by employing the measuring device 20; (iv) determining the length of the graft (soft tissue graft or BTB graft) based on the entire length of the sockets 81, 91 plus the intraarticular space between them, as measured by the device 20; and (v) securing the graft within the femoral and tibial tunnels (sockets) 81, 91.

Once the length of the graft is adequately determined, the graft is dimensioned according to the length indicated by the distance between the two sliding knots 22, 23 of the device 20 (Figure 5), and the dimensioned graft is subsequently prepared for retrograde fixation, for example. A continuous loop/button construct 30 (Figures 6 and 7) provided with a button 33, preferably of titanium alloy, and a continuous loop 35 attached to the button 33 may be employed for the graft fixation, and as described above.

Although the present invention has been described in connection with preferred embodiments, many modifications and variations will become apparent to those skilled in the art. While preferred embodiments of the invention have been described and illustrated above, it should be understood that these are exemplary of the invention and are not to be considered as limiting. Accordingly, it is not intended that the present invention be limited to the illustrated embodiments, but only by the appended claims.

## Claims

1. A measuring device (10, 20) for determining the length of a graft to be secured within a first bone tunnel and a second bone tunnel in combination with that graft, the measuring device (10, 20) comprising:
a flexible strand (11); and
two fixation devices (12, 13) affixed to the flexible strand (11);
wherein the two fixation devices are selected from the group consisting of a knot, a button and a washer; and the fixation devices (12, 13) are spaced apart from each other by a distance which is about equal to a sum of a first length of the first bone tunnel, a second length of the second bone tunnel, and a third length (D1) of an intraarticular space between the first bone tunnel and the second bone tunnel.

2. The measuring device (10, 20) of claim 1, wherein the two fixation devices (12, 13) are two sliding knots.

3. The measuring device (10, 20) of claim 1, wherein the two fixation devices (12, 13) are two buttons.

4. The measuring device (10, 20) of any preceding claim, wherein the at least one flexible strand (11) is a high strength suture.

5. The measuring device of any preceding claim, wherein the two fixation devices (12, 13) are two sliding knots and the graft is an ACL graft.

6. A measuring construct (10, 20) for determining the total length of a graft construct to be secured within a femoral tunnel and a tibial tunnel in combination with the graft construct, the measuring construct (10, 20) comprising:
a loop of flexible material (11) having a length about equal to a total length of the graft construct, the length being determined by adding a first length of the femoral tunnel, a second length of the tibial tunnel, and a third length (D1) representing the distance between the femoral tunnel and the tibial tunnel;
a first button (12) attached to the loop of flexible material (11); and
a second button (13) attached to the loop of flexible material (11).

7. The measuring construct of claim 6, wherein each of the first and second buttons (12, 13) comprises at least one opening configured to allow the loop to pass through it.

8. The measuring construct of claim 6 or 7, wherein the loop is formed of a suture material (11) comprising ultrahigh molecular weight polyethylene.

9. The measuring construct (10, 20) of any of claims 6 to 8, wherein at least one of the first and second buttons (12, 13) has an oblong configuration.

10. The measuring construct (10, 20) of any of claims 6 to 8, wherein at least one of the first and second buttons (12, 13) has a length of about 10mm to about 20mm.

11. The measuring construct (10, 20) of any of claims 6 to 9, wherein at least one of the first and second buttons (12, 13) has a width that is less than about 1mm narrower than the width of the femoral tunnel through which the button (12,13) is inserted and passed through.

12. The measuring construct (10, 20) of any of claims 6 to 10, wherein at least one of the first and second buttons (12, 13) is formed of a material selected from the group consisting of titanium, titanium alloy, polyethylene, PEEK and PLLA.

## Patentansprüche

1. Messvorrichtung (10, 20) zum Bestimmen der Länge eines Transplantats, das innerhalb eines ersten Knochentunnels und eines zweiten Knochentunnels in Kombination mit dem Transplantat sichert werden soll, wobei die Messvorrichtung (10, 20) Folgendes umfasst:
einen flexiblen Strang (11); und
zwei Fixierungsvorrichtungen (12, 13), die an dem flexiblen Strang (11) befestigt sind,
wobei die zwei Fixierungsvorrichtungen ausgewählt sind aus der Gruppe, besehend aus einem Knoten, einem Knopf und einer Unterlegscheibe; und die Fixierungsvornchtungen (12, 13) voneinander um eine Distanz beabstandet sind, die ungefähr gleich einer Summe einer ersten Länge des ersten Knochentunnels, einer zweiten Länge des zweiten Knochentunnels und einer dritten Länge (D1) eines Raums innerhalb von Gelenken zwischen dem ersten Knochentunnel und dem zweiten Knochentunnel ist.

2. Messvorrichtung (10, 20) nach Anspruch 1, wobei die zwei Fixierungsvorrichtungen (12, 13) zwei gleitende Knoten sind.

3. Messvorrichtung (10, 20) nach Anspruch 1, wobei die zwei Fixierungsvorrichtungen (12, 13) zwei Knöpfe sind.

4. Messvorrichtung (10, 20) nach einem der vorhergehenden Ansprüche, wobei der mindestens eine flexible Strang (11) ein hochfestes Nahtmaterial ist.

5. Messvorrichtung nach einem der vorhergehenden Ansprüche, wobei die zwei Fixierungsvorrichtungen (12, 13) zwei gleitende Knoten und das Transplantat ein ACL-Transplantat ist.

6. Messkonstrukt (10, 20) zum Bestimmen der gesamten Länge eines Transplantatkonstrukts, das innerhalb eines Oberschenkelknochen-Tunnels und eines Schienbein-Tunnels in Kombination mit dem Transplantatkonstrukt gesichert werden soll, wobei das Messkonstrukt (10, 20) Folgendes umfasst:
eine Schleife aus einem flexiblen Material (11) mit einer Länge, die ungefähr gleich einer gesamten Länge des Transplantatkonstrukts ist, wobei die Länge durch Addieren einer ersten Länge des Oberschenkelknochen-Tunnels, einer zweiten Länge des Schnienbein-Tunnels und einer dritten Länge (D1), die die Distanz zwischen dem Oberschenkelknochen-Tunnel und dem Schienbein-Tunnel darstellt, bestimmt ist,
einen ersten Knopf (12), der an der Schleife des flexiblen Materials (11) befestigt ist; und
einen zweiten Knopf (13), der an der Schleife des flexiblen Materials (11) befestigt ist.

7. Messvorrichtung nach Anspruch 6, wobei jeder des ersten und des zweiten Knopfs (12, 13) mindestens eine Öffnung umfasst, die konfiguriert ist, um zu ermöglichten, dass die Schleife durch sie verläuft.

8. Messkonstrukt nach Anspruch 6 oder 7, wobei die Schleife aus einem Nahtmaterial (11) gebildet ist, umfassend Polyethylen mit ultrahohem Molekulargewicht.

9. Messkonstrukt (10, 20) nach einem der Ansprüche 6 bis 8, wobei der mindestens eine des ersten und des zweiten Knopfs (12, 13) eine längliche Konfiguration aufweist.

10. Messkonstrukt (10, 20) nach einem der Ansprüche 6 bis 8, wobei mindestens einer des ersten und des zweiten Knopfs (12, 13) eine Länge von ungefähr 10 mm bis ungefähr 20 mm aufweist.

11. Messkonstrukt (10, 20) nach einem der Ansprüche 6 bis 9, wobei mindestens einer des ersten und des zweiten Knopfs (12, 13) eine Breite aufweist, die weniger als ungefähr 1 mm schmäler als die Breite des Oberschenkelknochen-Tunnels ist, durch den der Knopf (12, 13) eingeführt und geführt wird.

12. Messkonstrukt (10, 20) nach einem der Ansprüche 6 bis 10, wobei mindestens einer des ersten und des zweiten Knopfs (12, 13) aus einem Material gebildet ist, ausgewählt aus der Gruppe, bestehend aus Titan, Titanlegierung, Polyethylen, PEEK und PLLA.

## Revendications

1. Dispositif de mesure (10, 20) pour déterminer la longueur d'une greffe devant être fixée dans un premier tunnel osseux et un deuxième tunnel osseux en combinaison avec cette greffe, le dispositif de mesure (10, 20) comprenant :
un brin souple (11) ; et
deux dispositifs de fixation (12, 13) fixés sur le brin souple (11) ;
dans lequel les deux dispositifs de fixation sont sélectionnés dans le groupe constitué d'un noeud, d'un bouton et d'une rondelle, les dispositifs de fixation (12, 13) étant espacés l'un de l'autre d'une distance sensiblement égale à une somme d'une première longueur du premier tunnel osseux, d'une deuxième longueur du deuxième tunnel osseux, et d'une troisième longueur (D1) d'un espace intraarticulaire entre le premier tunnel osseux et le deuxième tunnel osseux.

2. Dispositif de mesure (10, 20) selon la revendication 1, dans lequel les deux dispositifs de fixation (12, 13) sont des noeuds coulissants.

3. Dispositif de mesure (10, 20) selon la revendication 1, dans lequel les deux dispositifs de fixation (12, 13) sont constitués par deux boutons.

4. Dispositif de mesure (10, 20) selon l'une quelconque des revendications précédentes, dans lequel le au moins un brin souple (11) est un fil de suture à résistance élevée.

5. Dispositif de mesure selon l'une quelconque des revendications précédentes, dans lequel les deux dispositifs de fixation (12, 13) sont constitués par deux noeuds coulissants, la greffe étant une greffe LCA.

6. Ensemble de mesure (10, 20) pour déterminer la longueur totale d'un ensemble de greffe destiné à être fixé dans un tunnel fémoral et un tunnel tibial en combinaison avec l'ensemble de greffe, l'ensemble de mesure (10, 20) comprenant :
une boucle de matériau souple (11) ayant une longueur sensiblement égale à une longueur totale de l'ensemble de greffe, la longueur étant déterminée en additionnant une première longueur du tunnel fémoral, une deuxième longueur du tunnel tibial, et une troisième longueur (D1) représentant la distance entre le tunnel fémoral et le tunnel tibial ;
un premier bouton (12) fixé sur la boucle de matériau souple (11) ; et
un deuxième bouton (13) fixé sur la boucle de matériau souple (11).

7. Ensemble de mesure selon la revendication 6, dans lequel chacun des premier et deuxième boutons (12, 13) comprend au moins une ouverture configurée de sorte à permettre le passage de la boucle.

8. Ensemble de mesure selon les revendications 6 ou 7, dans lequel la boucle est composée d'un matériau de suture (11) comprenant du polyéthylène à poids moléculaire ultra-élevé.

9. Ensemble de mesure (10, 20) selon l'une quelconque des revendications 6 à 8, dans lequel au moins un des premier et deuxième boutons (12, 13) a une configuration oblongue.

10. Ensemble de mesure (10, 20) selon l'une quelconque des revendications 6 à 8, dans lequel au moins un des premier et deuxième boutons (12, 13) a une longueur comprise entre environ 10 mm et environ 20 mm.

11. Ensemble de mesure (10, 20) selon l'une quelconque des revendications 6 à 9, dans lequel au moins un des premier et deuxième boutons (12, 13) a une largeur qui est moins d'environ 1 mm plus étroite que la largeur du tunnel fémoral à travers lequel le bouton (12, 13) est inséré et passé.

12. Ensemble de mesure (10, 20) selon l'une quelconque des revendications 6 à 10, dans lequel au moins un des premier et deuxième boutons (12, 13) est composé d'un matériau sélectionné dans le groupe constitué de titane, d'un alliage de titane, de polyéthylène, de PEEK et de PLLA.
